# EUROPEAN PATENT SPECIFICATION

(11) **EP 0 235 141 B1**
(45) Date of publication and mention of the grant of the patent: **25.08.1993**
(21) Application number: 85905747.3
(22) Date of filing: 26.11.1985
(51) Int. Cl.: G08B 21/00, G01J 1/44

(54) **ULTRAVIOLET RADIATION MONITORING DEVICE**
ÜBERWACHUNGSANORDNUNG FÜR ULTRAVIOLETTSTRAHLUNG
DISPOSITIF DE SURVEILLANCE DU RAYONNEMENT ULTRAVIOLET

(30) Priority: 26.11.1984 AU 8290/84
(43) Date of publication of application: 09.09.1987
(73) Proprietor: HEALTHTRONICS LIMITED, Osborne Park Western Australia (AU)
(72) Inventor: PEARSON, Anthony, Paul, Osborne Park, W.A. 6017 (AU)
(74) Representative: Matthews, Howard Nicholas
(86) International application number: AU8500294
(87) International publication number: WO8603319

(56) References cited:
- US-A- 4 428 050

## Description

This invention relates to an electromagnetic radiation monitoring dosimeter which is applicable to monitoring the amount of ultraviolet radiation falling upon a human body such as the exposed skin of a person and providing an indication to the person when an optimum amount of ultraviolet radiation has been absorbed over a period of time.

In the past two hundred years, many millions of Caucasians have emigrated from temperate and cool climates to warmer tropical climates throughout the world, *viz* America, South Africa, Australia, Israel, South East Asia ***et al***. In the light of the fact that it takes thousands of years for any biological change to occur in man, these immigrants and their hereditary offspring in future generations will very probably suffer from one form of skin cancer, or another. This is largely attributed to the absorption of an excessive amount of ultraviolet radiation by such persons in their biologically new environment, which may far exceed the level of ultraviolet radiation to which their bodies are accustomed, and, in fact are biologically capable of absorbing without detriment.

In Australia, particularly, it has become socially expedient for persons to acquire a suntan of their skins and so beachgoers and holiday makers spend a significant portion of their time sunbathing in the summer months. In most cases, such persons shall expose themselves to abnormal levels of ultraviolet radiation. Unfortunately, statistics show this activity to be extremely dangerous since it is considered to be a major contributing cause to skin cancer. In Australia alone, approximately six people die of skin cancer every week, and many tens of thousands more receive medical and surgical treatment for skin cancer related complaints every year. In the majority of cases, the afflicted person was totally ignorant of the fact that he or she could be exposed to a dangerous level of ultraviolet radiation by obtaining a bad sunburn on a minimal number of occasions.

It is an object of the invention to provide monitoring of electromagnetic radiation impinging a body and provide a sensorially perceptible alarm when the cumulative radiation to which the body is exposed attains a threshold level.

It is a preferred, although not essential, object of the invention to provide a dosimeter for monitoring ultraviolet radiation in accordance with a prescribed method which device is capable of being embodied as a self-contained, diminutive and portable unit, which can be carried on one's person during exposure to ultraviolet radiation.

Reference is made to US Patent No 4,229,733 which discloses a device for monitoring radiation from the sun and comprises a sensing means (12) for sensing radiation impinging a body and generate a sensing signal proportional to the magnitude of said impinging radiation; an integrating means (18, 14) to integrate said sensing signal in accordance with a first prescribed does function and generate an active signal (20) proportional to the magnitude of said integration; and control means (24) to monitor the relative magnitude of said active signal (20) and generate a sensorially perceptible signal (62) in response to said active signal (20) magnitude attaining a threshold level (26); wherein said first prescribed dose function is related to an admonished accumulative effect of said impinging radiation on said body and said threshold level (26) is related to the optimum cumulative radiation to which said body is desired to be exposed.

According to the invention there is provided a dosimeter for monitoring exposure of a human body to ultraviolet radiation comprising a sensor for sensing said radiation impinging a human body and to generate a sensing signal proportional to the intensity of said impinging radiation; and a first integrator adapted to integrate said sensing signal in accordance with a first prescribed dose function to generate an active signal representative of the magnitude of the accumulated dose of radiation received by said body having regard to the intrinsic tolerance of the body to said radiation; characterised by a second integrator adapted to integrate said sensing signal in accordance with a second prescribed dose function to generate a reference signal representative of the optimal dose of accumulated radiation to which said body is desired to be exposed; and a control circuit to monitor the relative magnitude of said active signal and said reference signal and generate a sensorially perceptible signal in response to the active signal magnitude attaining the reference signal magnitude.

In accordance with another aspect of the present invention, there is provided a method for monitoring exposure of a body to ultraviolet radiation comprising the steps of:
sensing ultraviolet radiation impinging a body;
generating a sensing signal proportional to the intensity of said impinging radiation;
performing a first integration of said sensing signal in accordance with a first prescribed dose function to provide an active signal representative of the accumulated dose radiation received by said body having regard to the intrinsic tolerance of the body to said radiation; characterised by
performing a second integration of said sensing signal in accordance with a second prescribed dose function to provide a reference signal representative of the optimal dose of accumulated radiation to which said body is desired to be exposed;
monitoring the relative magnitude of said active signal and said reference signal; and
generating a sensorially perceptible signal in response to said active signal magnitude attaining said reference signal magnitude.

In the accompanying drawings:
FIGURE 1 is a block diagram of a monitoring device disclosed for the purpose of reference;
FIGURE 2 is a circuit diagram of the monitoring device in accordance with an embodiment of the invention;
FIGURE 3 is a plan view of the physical arrangement of the monitoring device shown in Figure 2; and
FIGURE 4 is a side elevation of the arrangement shown in Figure 3.

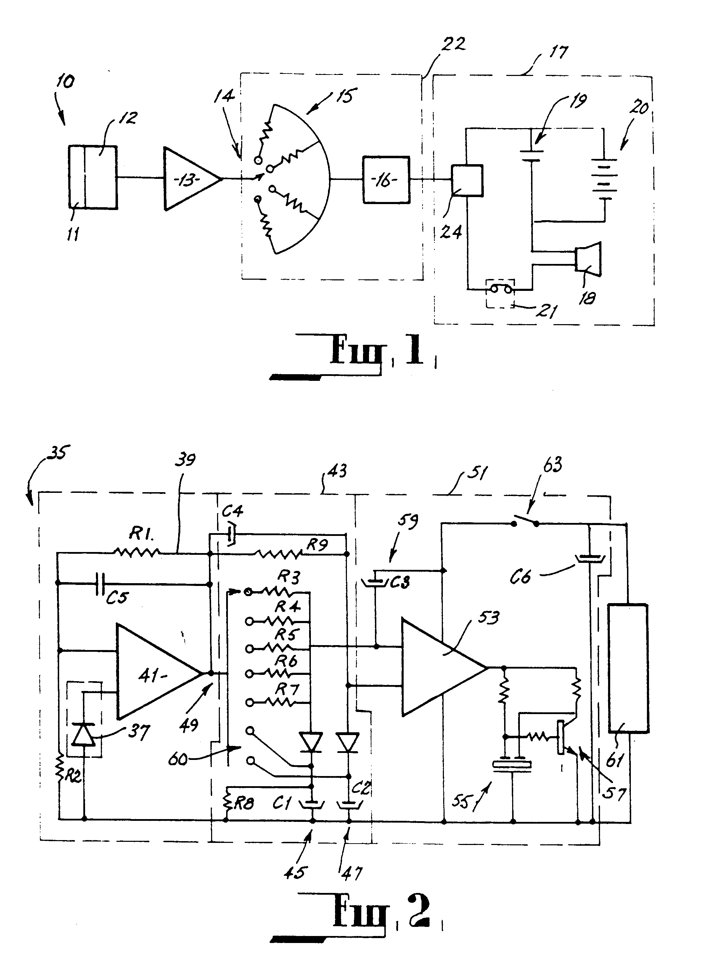

The embodiment shown in Figure 1 is an ultraviolet radiation monitoring device of diminutive size to enable the device to be worn on one's person.

With reference to Figs. 1, 3 and 4, the device comprises an outer casing 25 which encapsulates an electronic circuit, generally shown at Fig. 1, and appropriate transducers.

The casing 25 is provided with a window 27 which forms a surface 29 beneath which is located a sensing means 10 (see Fig. 1) comprising a filtering means 11 and detecting means 12. The filtering means 11 is a special ultraviolet filter which allows only ultraviolet radiation in the wavelength range of 290 nanometres to 400 nanometres to impinge the detecting means 12. This range is better known as including the UVA (320 nm to 400 nm) and the UVB (290 nm to 320 nm) types of ultraviolet radiation, the latter being the type that actually causes sunburn of the skin and the former being the type that causes wrinkling of the skin. The detector means 12 is a special type of transducer, being an enhanced silicon detector which provides a sensing signal in the form of an electric potential across a pair of terminals in response to impinging radiation. The magnitude of the signal is proportional to the instantaneous magnitude of the impinging radiation at any point in time. The output terminals of the detecting means 12 are in turn connected to a preamplifier 13 which amplifies the sensing signal created by the electric potential.

The output of the preamplifier 13 is connected to an integrating means 22 which includes a selector means 14, first resistor means 15, and memory means 16.

The selector means 14 is a single pole multi-throw switch, whereby a different value of first resistor means 15 is connected to respective terminals of the switch to accord with the designation of a first prescribed dose function of the integrating means. The first prescribed dose function is related to an admonished accummulative effect of the impinging radiation on the body of a person. To effect integration of the sensing signal, the memory means 16 includes a capacitor means, thus forming an R-C circuit. Accordingly the capacitor means stores charge dependent on the magnitude of the sensing signal at a rate prescribed by the time constant of the R-C circuit defining the first prescribed dose function.

The memory means also has a second resistor means (not shown) coupled in parallel with the capacitor means. The second resistor means provides a path for the capacitor means to discharge in the absence of the sensing signal magnitude. Accordingly the active signal magnitude is allowed to diminish at a rate determined by a first prescribed decay function which is defined by the time constant of the capacitor means and second resistor means. The first prescribed decay function is related to an admonished decay in the residual effect of previously impinging radiation on the body pursuant to said body being removed from said impinging radiation or vice versa.

The integrating means 22 consequently generates an active signal at its output proportional to the magnitude of the integration which output is connected to a control means 17.

The control means 17 effectively monitors the relative magnitude of the active signal and generates a sensorially perceptible signal in response to the active signal magnitude attaining a threshold level. The control means 17 comprises a comparator means and electronic switching means 24 which is open in response to the capacitor means having a stored charge, represented by the active signal magnitude, below a prescribed threshold level determined by a preset reference signal magnitude at the comparator means input, and is closed in response to the active signal magnitude exceeding the threshold level. The threshold level is related to the optimum cumulative radiation to which the body of a person is desired to be exposed. In the present embodiment, the electronic switching means is of the relay type which is interposed between a power source 19 and a transducing means 18 which generates the sensorially perceptible signal providing an alarm.

The power source 19 is a d.c. battery which is connected to a recharging circuit 20 comprising an encapsulated solar cell 26 disposed in juxtaposed relation to another window 28 of the casing. Serially connected between the transducing means and the switch is a photo electric switch 21. The switch is closed upon light impinging thereon and open in the absence of such, thereby providing additional means to disable operation of the device.

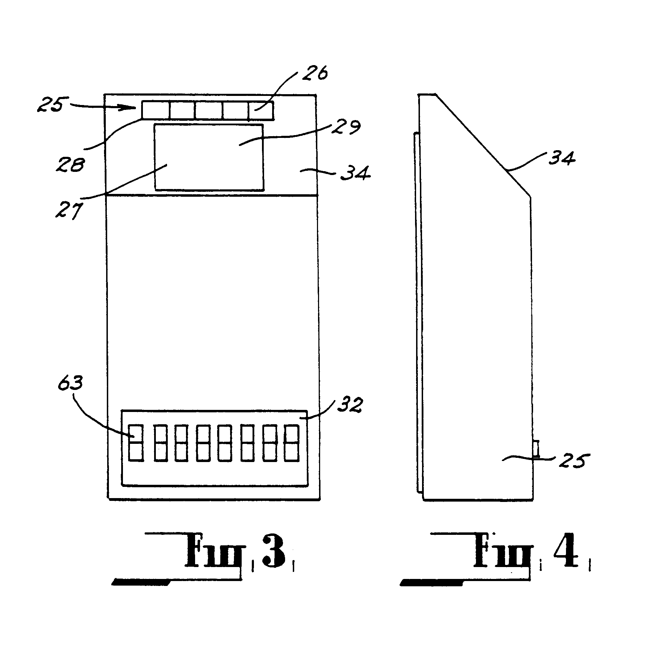

With reference to the physical arrangement of the device, as shown in Figs. 3 and 4, the selector means 14 is provided in the form of a multi-range switch 32. Accordingly the switch is provided with a number of positions which accord with the selection of a specific first resistor means 15 of the integrating means 22. The windows 27, 28 for the sensing means 10 and solar cell 26 are disposed on a sloping face 34 of the casing which is arranged at an oblique angle to the base and ends of the casing 25. This is to enable the windows to be exposed to impinging ultraviolet radiation in most attitudes that the casing would be likely to presume when carried by a user of the device. To achieve the best results, the casing is attached to a clothing item such as the hat of a user, where the sloping face 34 can be exposed to the ultraviolet radiation source for example the sun, continuously, irrespective of whether the user is vertical or horizontal to the ground.

A cover 30 is attached to the casing to enable it to be moved from an open to a closed position and vice versa. In the closed position, the cover is adapted to impede the passage of ultraviolet radition and light to the filtering means 11 and the photo electric switch 21 respectively, thereby disabling the device. In the open position, this impediment is removed and the device maybe enabled for operation.

Alternately an on/off switch is provided next to the skin type selector switches.

In operation the device receives ultraviolet radiation through the filtering means 11 which discriminates between the various frequencies of the impinging radiation and only allows the passage of radiation which causes sunburn and wrinkling, i.e. UVB and UVA, respectively through to the detecting means 12. The detecting means then produces a potential that is proportional in magnitude to the amount or magnitude of radiation applied thereto and thus generates a sensing signal which is amplified by the preamplifier and subsequently applied to the integrating means 22. The integration performed by the integrating means in accordance with the first prescribed dose function effectively provides monitoring of the amount of harmful radiation falling upon the device over a period of time. Accordingly when the active signal magnitude, representing the amount of charge stored within the memory means, exceeds the threshold level, the control means activates the transducing means 18 which generates a sensorially perceptible signal to the user or wearer of the device.

In the absence of incident radiation passing through the filtering means, the stored charge in the storage means will not be replenished and hence will effectively diminish in accordance with the first prescribed decay function.

In practice, the rate that the active signal increases and diminishes is set to correspond to the adaptability of the skin of a user or wearer to absorb ultraviolet radiation. Thus the device will be very sensitive on the user's first day of exposure to ultraviolet radiation, by selecting a first resistor means 15 to combine with the capacitor means of the memory means 16 to provide a first prescribed dose function imposing a relatively fast rate of increase of the active signal subject to the presence of the sensing signal. Accordingly the alarm will be activated after only a relatively short time of exposure to the sun. On subsequent days, as the Melanin in the skin of the user builds up, another first resistor means can be selected which combines with the capacitor means to provide a first prescribed dose function imposing a slower rate of increase in the active signal. Accordingly the alarm will be activated after a longer period of time of re-exposure of the user and the device to the radiation. Similarly as the skin of the user is progressively tanned, other first resistor means can be selected to provide longer delays. It should be noted however, that on the user sheltering himself from the sun, the active signal diminishes at a rate imposed by the first prescribed decay function. Thus the memory means operates to cater for situations where the user may be re-exposed to the ultraviolet radiation after only a short period of time has elapsed. In such cases the capacitor means would not be fully discharged and thus will cause the active signal to approach the threshold level after a very much shorter period of time than before. This is commensurate with the adaptability of the user's skin to absorb further radiation after only a short relaxation from previous exposure, which in most cases is not very great. The provision of the selector means allows the device to be adjusted to match the different types of skin sensitivity of users of the device. In addition, the selector means allows a particular user to dampen the sensitivity of the device if the user has already acquired a partial tan before using the device, thereby increasing the allowable time of exposure of the skin to impinging ultraviolet radiation.

An embodiment of the present invention will now be described with reference to Figures 2, 3 and 4 of the drawings.

As in the preceding reference embodiment, the present device generally comprises a sensing means, integrating means and control means. The electronic circuitry of the sensing means is shown generally at 35 and includes a filtering means (not shown) and detector means 37 as described in the preceding embodiment.

The output of the detector means 37 is connected to the input of the preamplifier 39 which is in the form of an operational amplifier. As shown in Figure 2, the operational amplifier is connected in the non-inverting amplifier mode, wherein the output of the detecting means 37 is connected to the non-inverting input of the operational amplifier 41 and the gain of the amplifier is provided by means of resistors R1 and R2. For the present application, these resistors are set to provide a gain of approximately 80. Coupled in parallel to resistor R1 in the feedback loop of the amplifier is a capacitor C5, which forms a delay means, the function of which shall be described in more detail later.

The output of the preamplifier 39 is connected to the integrating means 43, which generally comprises a memory means 45, a further memory means 47 and a selector means 49.

The selector means 49 comprises a plurality of differently valued first resistor means R3 to R7. These resistor means are arranged in order of increasing value and are arranged so that a single resistor can be coupled in series with the memory means 45 and the output of the preamplifier 39. The memory means 45 comprises a capacitor means C1 which combines with the particular selected first resistor means to provide a time constant which defines a first prescribed dose function. This first prescribed dose function is of a similar kind as defined in the first embodiment, but when considered in the context of the present embodiment, is determined on a slightly different conceptual basis than the latter. This shall be discussed in more detail later.

Coupled in parallel to the capacitor means C1 is a second resistor means R8 which provides another time constant for the discharge of the capacitor means, so defining a first prescribed decay function. The first prescribed decay function is substantially identical to that described in the preceding embodiment.

The further memory means 47 comprises a further capacitor means C2 which is also connected to the output of the preamplifier, but via a third resistor means R9. The third resistor means R9 combines with the capacitor means C2 to provide a further time constant defining in part, a second prescribed dose function.

Coupled in parallel to the third resistor means R9 is a bypass capacitor means C4 which also combines with the further capacitor means C2 to define the other part of the secondprescribed dose function.

Accordingly, the second prescribed dose function consists of an initial and a subsequent component, wherein the initial component is defined by the combination of the bypass capacitor means C4 connected in series when the further capacitor means C2, and the subsequent component is provided by the combination of the third resistor means R9 connected in series with the further capacitor means C2. The function of these components is arranged such that the initial component provides an initial integration of a signal appearing at the output of the preamplifier 39 at a greater rate than the first prescribed dose function, and the subsequent component provides a subsequent integration at a lesser rate than the first prescribed dose function.

The respective outputs of the capacitor means C1 and C2 are connected via diodes D1 and D2 respectively to the inputs of the control means 51. These diodes prevent the discharge of the respective capacitor means back through the input circuit of the integrating means. Thus, in the case of capacitor means C2, discharge of the same is only provided by its innate leakage which defines a second prescribed decay function. Obviously, the second prescribed decay function provides a rate of decay significantly less than the rate of decay as provided by the first prescribed decay function for the capacitor C1.

The integrating means is provided with a further switching means 60 which is incorporated into the selector means 49 to enable independent discharging or resetting of the capacitor means C1 and C2 by the user. This is provided by directly connecting the output of the preamplifier to a capacitor means selected by the further switching means 60, which effectively sinks the stored charge within the selected capacitor means.

The control means 51 comprises comparator means 53, transducing means 55 and electronic switching means 57. The comparator means 53 has its inputs 5 and 6 connected to the outputs of the memory means 45 and further memory means 47 respectively, and its own output 7 connected serially to the transducing means 57.

The electronic switching means is in the form of a transistor connected in the common emitter configuration. the base and collector terminals of the transistor are both driven by the output 7 of the comparator means and so switched on only in response to an active output signal being provided by the comparator means. The transducing means 55 is provided by the comparator means. The transducing means 55 is in the form of a piezzo-electric crystal having an integrated oscillating circuit provided therein. The electronic switching means 57 is connected intermediate the comparator means 53 and transducing means 55 to enable and disable the piezzo-electric crystal and oscillating means in response to the condition of the comparator means. On activation of the piezzo-electric crystal the oscillating means functions to drive the crystal at a prescribed frequency enabling the same to produce a sensorially perceptible signal, particularly perceptible to the aural senses of a person using the device.

The control means also includes a triggering means 59 in the form of an actuating capacitor means C3 coupled between a power source 61 for the device and the output of the memory means 45. The triggering means 59 is provided to incite generation of the sensorially perceptible signal by the control means for a short period on initially supplying power to the device. The operation of this shall be described in more detail later.

The power source 61 is in the form of a solar cell providing power to the device via a switch 63. To facilitate operation of the solar cell, a storage capacitor C6 is connected across the power terminals thereof to sustain the output voltage of the cell.

With reference to the physical configuration of the device, as shown in Figs. 3 and 4, the arrangement is substantially identical to that of the preceding embodiment. Thus, the filtering means and detecting means 37 are provided beneath the window 27, the solar cell 61 is provided beneath the window 28, and the selector means 49 is provided by way of the multi-range switch 32 of the device. In addition, the power switch 63 can be provided separately on the casing as shown at 65, or incorporated into a position provided on the multi-range switch, whereby the device may be turned off or on by moving the switch into or out of this position respectively.

The operation of the device shall now be described.

The filtering means and detecting means of the sensing means 35 operates substantially the same as in the preceding embodiment. A sensing signal is provided at the output of the detecting means 37 which is input to the non-inverting input of the operational amplifier 41 and is subsequently amplified by the preamplifier arrangement 39 to provide a sensing signal input to the integrating means 43.

The integrating means 43 essentially integrates this sensing signal via two parallel paths which are defined respectively by the memory means 45 and further memory means 47. The first path integrates the sensing signal in accordance with the first prescribed dose function and generates an active signal at the output of the memory means 45. The second path integrates the sensing signal in accordance with the second prescribed dose function and generates a reference signal at the output of the further memory means 47. As previously described, the rate of integration of the further memory means 47 is initially greater than that of the memory means 45 since on the initial generation of the sensing signal the bypass capacitor means C4 functions to bypass the third resistor means R9 associated therewith and so enabling the further capacitor means C2 to charge very rapidly. This rapid charge will continue until the additive charge of the further capacitor means C2 and bypass capacitor means C4 approaches the magnitude of the sensing signal, whereon additional accumulative charge will be diverted through resistor R9. This diversion subsequently has the effect of the further capacitor means C2 charging at a subsequent rate prescribed by the subsequent component of the second prescribed dose function. Thus, effectively the further capacitor means C2 charges at an initial rate which is greater than the charging rate of capacitor means C1, so providing a reference signal magnitude that is initially higher than that of the active signal. However, on subsequent charging, the further capacitor means C2 will proceed at a charging rate slower than that of the capacitor means C1. Thus after a time which is prescribed by the selected values of the first resistor means R3 to R7, third resistor means R9 and the capacitor means C1, C2 and C, the active signal magnitude shall eventually attain the threshold level set by the reference signal magnitude.

The comparator means 53 of the control means 51 effectively monitors the relative magnitudes of the active signal and reference signal, so that during the period that the active signal is less than the reference signal the output of the comparator means is deactivated, and on the active signal magnitude attaining the threshold level set by the reference signal magnitude, the comparator means output will be activated. On activation of the comparator means output, the electronic switching means will be turned on enabling the operation of the transducing means 55.

It should be noted that an important feature and difference of the present embodiment over the preceding embodiment, is that due to the integrating means 43 providing a rising reference signal and thus rising threshold level, which effectively rises progressively at two different rates in accordance with the second prescribed dose function, the first prescribed dose function can be chosen having consideration to fewer parameters than is required in the preceding embodiment. Therefore the first prescribed dose function need be only chosen with respect to a particular person's skin type and need not be changed on exposure to the impinging ultraviolet radiation by the user on different days as the user's tolerance to radiation increases.

This effect is achieved principally by providing a reference signal magnitude which automatically increases on increased exposure of the device to ultraviolet radiation, and having the further capacitor means C2 diminishing the reference signal magnitude only in response to the second prescribed decay function, in contrast to the capacitor means C1 diminishing the active signal magnitude in accordance with the first prescribed decay function. Moreover the first and second decay functions are set so that the capacitor means C1 discharges fully overnight, whereas the further capacitor means C2 discharges fully only over a period of approximately 1 week. Thus, on a person exposing themselves to ultraviolet radiation on successive days for the prescribed periods of time as determined by the device, the further capacitor means C2 will have some residual charge after a previous days use of the device, thereby providing a reference signal at a higher level of magnitude on commencing use of the device than the previous day. Accordingly, the capacitor means C1 will take a longer period of time to attain the level of the reference signal and so by selecting suitable component values, the integrating means can be arranged to simulate the build-up of Melanin in the skin of the user of the device and thus allow for safe prolonged periods of exposure to ultraviolet radiation ensuring the safe progressive tanning of the user's skin.

Therefore, it is only necessary for a user to select the appropriate position of the multi-range switch 32 to match his particular skin type, whereinafter by following the prescribed method of use of the device, the allowable exposure of the user to ultraviolet radiation will automatically be compensated for by the device on progressive exposure to the radiation.

On initial switch-on of the device, the triggering means 59 operates to provide an active signal which is initially higher in magnitude than the reference signal for a very short period of time, and which is sufficient to incite generation of the sensorially perceptible signal for a short period. This period will only last until the actuating capacitor C3 charges up to the potential of the power source supplied to the device. To enable this action to occur without detriment to the subsequent operation of the integrating means 43, the delay means operates to negative the function of the sensing means for the duration of the period that the active signal exceeds the level of the reference signal. This is achieved by the bypass capacitor C4 negating the effect of the feedback resistor R1 for a short period of time thus suppressing the gain of the preamplifier until such time as C4 charges to divert the feedback current through feedback resistor R1. By the time that the gain of the preamplifier is reinstated, the effect of the triggering means 59 would have been completed.

An incidental feature of the device is that the comparator means 53 is provided with further triggering means in the form of an intrinsic capacitance at each of its inputs (not shown). This capacitance is inherent within the circuit as configured within an integrated circuit arrangement. Accordingly, whenever the sensing signal which is input to the integrating means 43 is absent concurrent with power being supplied to the device, as may arise when the detecting means 37 is obstructed from impinging radiation by some impediment, the intrinsic capacitance discharges back through the integrating means via the reference and active lines respectively. The time constants for the respective discharge lines will conseqently be determined by the third and first prescribed dose functions respectively. Since the bypass capacitor means C4 determines the initial component of the second prescribed dose function, the intrinsic capacitance will be allowed to rapidly discharge initially by bypassing the third resistor means R9, in contrast to the discharge of the intrinsic capacitance via the first resistor means R3-R7 of the active line. Consequently, the reference signal will initially rapidly diminish in comparison to the active signal causing the reference level to be attained by the latter, and so activating the comparator means output enabling the operation of the transducing means 55.

Thus the sensorially perceptible signal will be generated whenever impinging radiation is intentionally or unintentionally obstructed from the detecting means by some impediment. For example by a fly or suntan lotion landing on the device covering the sensing window 27.

The particular advantages that are capable of being provided by the device as described in any of the preceding embodiments include:-
(1) guarding against skin cancer and premature skin ageing;
(2) recognising different skin types and automatically setting an ultraviolet dose limit accordingly;
(3) remembering the ultraviolet dose of previous days and modifying current dose accordingly;
(4) matching the Melanin build up in the user's skin day by day so giving maximum safety in addition to a tan and with or without a skin protection cream;
(5) the unit can be completely sealed and is self-contained and so can be worn on a hat or other apparel such as bathing costumes etc. of a person even when swimming, thereby continuously operating to monitor the dose of ultraviolet radiation received;
(6) the device is ideal for sunbathing by providing a means to inform the user of excessive exposure to the sun at any time the user is exposed; and
(7) the device can be used in any number of situations where persons may be working on road construction, building sites, farming, oil and mineral exploration, watching sporting events, gardening, washing the car etc, with or without a skin protection cream.

It should be appreciated that the scope of the present invention is not limited to the scope of the embodiment herein described. Particularly the switching means is not limited to the use of a cover and photo electric switch, but may consist of other suitable means. In addition, the sensorially perceptible alarm, is not necessarily limited to an aural signal but may also include a visual indicator to provide a visual signal. Furthermore, the power source is not necessarily limited to a solar cell but may consist of any other suitable device, such as a replaceable battery.

## Claims

1. A dosimeter for monitoring exposure of a human body to ultraviolet radiation comprising a sensor (35) for sensing said radiation impinging a human body and to generate a sensing signal proportional to the intensity of said impinging radiation; and a first integrator (43) adapted to integrate said sensing signal in accordance with a first prescribed dose function to generate an active signal representative of the magnitude of the accumulated dose of radiation received by said body having regard to the intrinsic tolerance of the body to said radiation; characterised by a second integrator adapted to integrate said sensing signal in accordance with a second prescribed dose function to generate a reference signal representative of the optimal dose of accumulated radiation to which said body is desired to be exposed; and a control circuit (51) to monitor the relative magnitude of said active signal and said reference signal and generate a sensorially perceptible signal in response to the active signal magnitude attaining the reference signal magnitude.

2. A dosimeter as claimed in claim 1, characterised in that said first integrator includes a first memory (45) associated therewith to store said active signal magnitude and allow said active signal magnitude to diminish in accordance with a first prescribed decay function in response to a reduction in said sensing signal magnitude, said first prescribed decay function being related to a predetermined decay in the immediate effect of said accumulated dose on said body pursuant to said body being removed from said impinging radiation or ***vice versa***, and said second integrator has a second memory (47) to store said reference signal magnitude and allow the reference signal magnitude to diminish in accordance with a second prescribed decay function in response to a reduction in said sensing signal magnitude, said second prescribed decay function being related to a predetermined decay in the delayed effect of said accumulated dose on said body pursuant to said body being removed from said impinging radiation or ***vice versa***, whereby the rate of decay in accordance with said second prescribed decay function is less than the rate of decay in accordance with said first prescribed decay function.

3. A dosimeter as claimed in claim 1, characterised in that said second prescribed dose function comprises initial and subsequent components, said initial component providing an initial integration at a greater rate than said first prescribed dose function, and said subsequent component providing a subsequent integration at a lesser rate than said first prescribed dose function.

4. A dosimeter as claimed in claim 1, characterised in that said first integrator (43) includes a selector including said first resistor to select different first prescribed dose functions dependent upon the tolerance of said body in connection with which said dosimeter is intended to be used.

5. A dosimeter as claimed in claim 2, characterised in that said first memory (45) comprises a first capacitor (C1) and said first function is determined by first resistors (R3-R7) coupled in series with said capacitor (C1) to form a first series R-C charge circuit.

6. A dosimeter as claimed in claim 5, characterised in that said first integrator (43) includes a selector to select different first prescribed dose functions dependent upon the tolerance of said body in connection with which said dosimeter is intended to be used, said selector comprising a plurality of differently valued first resistors (R3-R7) and a switch (60) to selectively couple a single first resistor (R3-R7) to said capacitor (C1) at any one time.

7. A dosimeter as claimed in claim 3, characterised in that said second memory (47) comprises a second capacitor (C2) and the initial component of said second prescribed dose function is determined by a bypass capacitor (C4) coupled in series to said second capacitor (C2) and the subsequent component of said second prescribed dose function is determined by a second resistor (R9) coupled in series with said third capacitor (C3) and in parallel with said bypass capacitor (C4).

8. A dosimeter as claimed in claim 7, characterised in that said first capacitor (C1) and said second capacitor (C2) are arranged so that said first and second prescribed dose functions thereof are determined by the innate leakage of said first capacitor (C2) and said second capacitor (C2) respectively.

9. A dosimeter as claimed in claim 7, characterised in that said second memory (47) is provided with resetting means to delete said reference signal magnitude therein upon said body being removed from said impinging radiation or ***vice versa***.

10. A dosimeter as claimed in claim 6, characterised in that said second memory (47) comprises a second capacitor (C2) connected to said second selector for specific first prescribed dose functions to provide a path for discharging said second capacitor substantially instantaneously upon said body being removed from said impinging radiation or ***vice*** ***versa***.

11. A dosimeter as claimed in claim 1, characterised in that said control circuit (51) includes a transducer (55) to generate said sensorially perceptible signal, and an electronic switch (52) to activate said transducer in response to said active signal magnitude attaining said reference signal magnitude.

12. A dosimeter as claimed in claim 1, characterised in that said control circuit includes a trigger circuit (59) to incite generation of said sensorially perceptible signal momentarily upon power being supplied to said dosimeter to indicate that said dosimeter is operational.

13. A dosimeter as claimed in claim 12, characterised in that said control circuit includes a comparator (53) which has the active signal output and reference signal output coupled to its inputs and said trigger circuit comprises an actuating capacitor coupled between a power source for the dosimeter and the active signal output of said first integrator, such that on supplying power to said dosimeter, said active signal magnitude will rapidly attain said reference signal magnitude and subsequently return to a nominal magnitude specified by said first integrator consequential to said actuating capacitor becoming fully charged.

14. A dosimeter as claimed in claim 12, characterised in that said sensor includes delay means to negate its function substantially during the momentary generation of said sensorially perceptible signal.

15. A dosimeter as claimed in claim 1, characterised by a further trigger circuit adapted to incite generation of said sensorially perceptible signal in the absence of said sensing signal after power has been supplied to said dosimeter.

16. A dosimeter as claimed in claim 15, characterised in that said further trigger circuit incites generation of said sensorially perceptible signal momentarily when both said power and said sensing signal simultaneously rapidly reduce in magnitude or become absent.

17. A method for monitoring exposure of a body to ultraviolet radiation comprising the steps of:
sensing ultraviolet radiation impinging a body;
generating a sensing signal proportional to the intensity of said impinging radiation;
performing a first integration of said sensing signal in accordance with a first prescribed dose function to provide an active signal representative of the accumulated dose radiation received by said body having regard to the intrinsic tolerance of the body to said radiation; characterised by
performing a second integration of said sensing signal in accordance with a second prescribed dose function to provide a reference signal representative of the optimal dose of accumulated radiation to which said body is desired to be exposed;
monitoring the relative magnitude of said active signal and said reference signal; and
generating a sensorially perceptible signal in response to said active signal magnitude attaining said reference signal magnitude.

18. A method as claimed in claim 17, characterised by the step of storing said active signal magnitude to diminish in accordance with a first prescribed decay function in response to a reduction in said sensing signal magnitude, said first prescribed decay function being related to a predetermined decay in the immediate effect of said accumulated dose on said body pursuant to being removed from said impinging radiation or ***vice versa***, and storing said reference signal magnitude and allowing the reference signal magnitude to diminish in accordance with a second prescribed decay function in response to a reduction in said sensing signal magnitude, said second prescribed decay function being related to a predetermined decay in the delayed effect of said accumulated dose on said body pursuant to said body being removed from said impinging radiation or ***vice versa***, whereby the rate of decay of said second prescribed decay function is less than the rate of decay of said first prescribed decay function.

19. A method as claimed in claim 18, characterised in that said second prescribed dose function comprises initial and subsequent components, said initial component providing an initial integration at a greater rate than said first prescribed dose function, and said subsequent component providing a subsequent integration at a lesser rate than said first prescribed dose function.

20. A method as claimed in claim 17, characterised by the step of inciting generation of said sensorially perceptible signal momentarily upon power being supplied to monitor said ultraviolet radiation.

21. A method as claimed in claim 20, characterised by the step of negating the sensing step substantially during the momentary generation of said sensorially perceptible signal.

22. A method as claimed in claim 17, characterised by the step of filtering some of the radiation to which the body is exposed to only allow radiation in the ultraviolet region of the electromagnetic spectrum to be sensed.

23. A method as claimed in claim 17, characterised by the step of inciting generation of said sensorially perceptible signal in the absence of said sensing signal after supplying power to monitor said ultraviolet radiation.

24. A method as claimed in claim 23, characterised in that said sensorially perceptible signal is momentarily generated when both said power and said sensing signal are simultaneously rapidly reduced in magnitude or become absent.

## Patentansprüche

1. Dosismeßgerät zur Überwachung der Belastung eines menschlichen Körpers mit ultravioletter Strahlung mit einem Meßfühler (35) zum Messen der besagten auf einen menschlichen Körper auftreffenden Strahlung und zum Erzeugen eines zur Intensität der besagten auftreffenden Strahlung proportionalen Meßsignals; und einem ersten Integrator (43) zur Integrierung des besagten Meßsignals nach einer ersten vorgeschriebenen Dosisfunktion zur Erzeugung eines aktiven, die Höhe der vom besagten Körper aufgenommenen kumulierten Strahlungsdosis darstellenden Signals unter Berücksichtigung der Eigentoleranz des Körpers für die besagte Strahlung; gekennzeichnet durch einen zweiten Integrator zur Integrierung des besagten Meßsignals nach einer zweiten vorgeschriebenen Dosisfunktion zur Erzeugung eines Bezugssignals, das die optimale Dosis kumulierter Strahlung darstellt, mit der der besagte Körper belastet werden soll; und einen Steuerkreis (51) zur Überwachung der relativen Höhe des besagten Aktivsignals und besagten Bezugssignals und zum Erzeugen eines sensorisch wahrnehmbaren Signals als Reaktion darauf, daß die Höhe des Aktivsignals die Höhe des Bezugssignals erreicht.

2. Dosismeßgerät nach Anspruch 1, dadurch gekennzeichnet, daß der besagte erste Integrator einen ersten damit verbundenen Speicher (45) enthält, um die besagte Höhe des Aktivsignals zu speichern und die Höhe des besagten Aktivsignals als Reaktion auf eine Verringerung der Höhe des besagten Meßsignals nach einer ersten vorgeschriebenen Abklingfunktion abnehmen zu lassen, wobei die besagte erste vorgeschriebene Abklingfunktion mit einem vorbestimmten Abklingen der Sofortwirkung der besagten kumulierten Dosis auf den besagten Körper nach Entfernung des besagten Körpers aus der besagten auftreffenden Strahlung oder umgekehrt in Beziehung steht, und der besagte zweite Integrator einen zweiten Speicher (47) besitzt, um die besagte Bezugssignalhöhe zu speichern und die Bezugssignalhöhe als Reaktion auf eine Verringerung der besagten Meßsignalhöhe nach einer zweiten vorgeschriebenen Abklingfunktion abnehmen zu lassen, wobei die besagte zweite vorgeschriebene Abklingfunktion mit einem vorbestimmten Abklingen der verzögerten Wirkung der besagten kumulierten Dosis auf den besagten Körper nach Entfernung des besagten Körpers aus der besagten auftreffenden Strahlung oder umgekehrt in Beziehung steht, wodurch die Abklinggeschwindigkeit nach der besagten zweiten vorgeschriebenen Abklingfunktion geringer als die Abklinggeschwindigkeit nach der besagten ersten vorgeschriebenen Abklingfunktion ist.

3. Dosismeßgerät nach Anspruch 1, dadurch gekennzeichnet, daß die besagte zweite vorgeschriebene Dosisfunktion erste und nachfolgende Komponenten umfaßt, wobei die besagte Erstkomponente eine Erstintegrierung mit größerer Geschwindigkeit als besagte erste vorgeschriebene Dosisfunktion bereitstellt und die besagte Folgekomponente eine nachfolgende Integrierung mit einer geringeren Geschwindigkeit als die besagte erste vorgeschriebene Dosisfunktion bereitstellt.

4. Dosismeßgerät nach Anspruch 1, dadurch gekennzeichnet, daß der besagte erste Integrator (43) eine Wählvorrichtung einschließlich des besagten ersten Widerstandes zum Auswählen unterschiedlicher erster vorgeschriebener Dosisfunktionen in Abhängigkeit von der Toleranz des besagten Körpers enthält, im Zusammenhang mit dem das besagte Dosismeßgerät benutzt werden soll.

5. Dosismeßgerät nach Anspruch 2, dadurch gekennzeichnet, daß der besagte erste Speicher (45) einen ersten Kondensator (C1) umfaßt und die besagte erste Funktion durch erste in Serie mit dem besagten Kondensator (C1) geschaltete Widerstände (R3-R7) zum Bilden eines ersten R-C-Serienladekreises bestimmt ist.

6. Dosismeßgerät nach Anspruch 5, dadurch gekennzeichnet, daß der besagte erste Integrator (43) eine Wählvorrichtung zum Auswählen unterschiedlicher erster vorgeschriebener Dosisfunktionen in Abhängigkeit von der Toleranz des besagten Körpers enthält, im Zusammenhang mit dem das besagte Dosismeßgerät benutzt werden soll, wobei die besagte Wählvorrichtung mehrere unterschiedlich bewertete erste Widerstände (R3-R7) und einen Schalter (60) zum gezielten Anschalten eines einzelnen ersten Widerstandes (R3-R7) an den besagten Kondensator (C1) zu einer jeweiligen Zeit umfaßt.

7. Dosismeßgerät nach Anspruch 3, dadurch gekennzeichnet, daß der besagte zweite Speicher (47) einen zweiten Kondensator (C2) umfaßt und die Erstkomponente der besagten zweiten vorgeschriebenen Dosisfunktion durch einen in Serie mit dem besagten zweiten Kondensator (C2) geschalteten Überbrückungskondensator (C4) bestimmt wird und die Folgekomponente der besagten zweiten vorgeschriebenen Dosisfunktion durch einen zweiten in Serie mit dem besagten dritten Kondensator (C3) und parallel mit dem besagten Überbrückungskondensator (C4) geschalteten Widerstand (R9) bestimmt wird.

8. Dosismeßgerät nach Anspruch 7, dadurch gekennzeichnet, daß der besagte erste Kondensator (C1) und der besagte zweite Kondensator (C2) so angeordnet sind, daß deren besagte erste und zweite vorgeschriebene Dosisfunktionen durch die Eigenstreuung des besagten ersten Kondensators (C2) durch die Eigenstreuung des besagten ersten Kondensators (C1) bzw. des besagten zweiten Kondensators (C2) bestimmt werden.

9. Dosismeßgerät nach Anspruch 7, dadurch gekennzeichnet, daß der besagte zweite Speicher (47) mit Rücksetzmitteln zum Löschen der darin enthaltenen besagten Bezugssignalhöhe bei Entfernung des besagten Körpers aus der besagten auftreffenden Strahlung oder umgekehrt versehen ist.

10. Dosismeßgerät nach Anspruch 5, dadurch gekennzeichnet, daß der besagte zweite Speicher (47) einen mit der besagten zweiten Wählvorrichtung verbundenen zweiten Kondensator (C2) für spezifische erste vorgeschriebene Dosisfunktionen umfaßt, um einen Weg zum im wesentlichen sofortigen Entladen des besagten zweiten Kondensators bei Entfernung des besagten Körpers aus der besagten auftreffenden Strahlung oder umgekehrt bereitzustellen.

11. Dosismeßgerät nach Anspruch 1, dadurch gekennzeichnet, daß der besagte Steuerkreis (51) einen Wandler (55) zur Erzeugung des sensorisch wahrnehmbaren Signals enthält, und einen elektronischen Schalter (52) zur Betätigung des besagten Wandlers als Reaktion darauf, daß die besagte Aktivsignalhöhe die besagte Bezugssignalhöhe erreicht.

12. Dosismeßgerät nach Anspruch 1, dadurch gekennzeichnet, daß der besagte Steuerkreis einen Triggerkreis (59) zur momentanen Anregung der Erzeugung des besagten sensorisch wahrnehmbaren Signals bei Anlegen von Strom an das besagte Dosismeßgerät enthält, um anzuzeigen, daß das besagte Dosismeßgerät betriebsbereit ist.

13. Dosismeßgerät nach Anspruch 12, dadurch gekennzeichnet, daß der besagte Steuerkreis einen Vergleicher (53) enthält, dessen Aktivsignalausgang und Bezugssignalausgang mit seinen Eingängen verbunden sind und der besagte Triggerkreis einen zwischen eine Stromquelle für das Dosismeßgerät und den Aktivsignalausgang des besagten ersten Integrators geschalteten Betätigungskondensator umfaßt, so daß bei Anlegen von Strom an das besagte Dosismeßgerät die besagte Aktivsignalhöhe schnell die besagte Bezugssignalhöhe erreicht und danach auf eine nach voller Aufladung des besagten Betätigungskondensators vom besagten ersten Integrator angegebene Nennhöhe zurückkehrt.

14. Dosismeßgerät nach Anspruch 12, dadurch gekennzeichnet, daß der besagte Meßfühler Verzögerungsmittel enthält, um seine Funktion während der momentanen Erzeugung des besagten sensorisch wahrnehmbaren Signals im wesentlichen zu negieren.

15. Dosismeßgerät nach Anspruch 1, gekennzeichnet durch einen weiteren Triggerkreis zur Anregung der Erzeugung des besagten sensorisch wahrnehmbaren Signals in Abwesenheit des besagten Meßsignals nach Anlegen von Strom an das besagte Dosismeßgerät.

16. Dosismeßgerät nach Anspruch 15, dadurch gekennzeichnet, daß der besagte weitere Triggerkreis momentan die Erzeugung des besagten sensorisch wahrnehmbaren Signals anregt, wenn sowohl der besagte Strom als auch das besagte Meßsignal gleichzeitig schnell an Höhe verlieren oder verschwinden.

17. Verfahren zur Überwachung der Belastung eines Körpers mit ultravioletter Strahlung mit folgenden Schritten:
Messen von auf einen Körper auftreffender ultravioletter Strahlung;
Erzeugen eines zur Intensität der besagten auftreffenden Strahlung proportionalen Meßsignals;
Durchführen einer ersten Integrierung des besagten Meßsignals nach einer ersten vorgeschriebenen Dosisfunktion zur Bereitstellung eines die vom besagten Körper aufgenommene kumulierte Strahlungsdosis darstellenden Aktivsignals unter Berücksichtigung der Eigentoleranz des Körpers für die besagte Strahlung; gekennzeichnet durch
Durchführen einer zweiten Integrierung des besagten Meßsignals nach einer zweiten vorgeschriebenen Dosisfunktion zur Bereitstellung eines Bezugssignals, das die optimale Dosis kumulierter Strahlung darstellt, mit der der besagte Körper belastet werden soll;
Überwachen der relativen Höhe des besagten Aktivsignals und besagten Bezugssignals; und
Erzeugen eines sensorisch wahrnehmbaren Signals als Reaktion darauf, daß die besagte Aktivsignalhöhe die besagte Bezugssignalhöhe erreicht.

18. Verfahren nach Anspruch 17, gekennzeichnet durch den Schritt des Speicherns der besagten Aktivsignalhöhe zum Abnehmen nach einer ersten vorgeschriebenen Abklingfunktion als Reaktion auf eine Verringerung der besagten Meßsignalhöhe, wobei die besagte erste vorgeschriebene Abklingfunktion mit einem vorbestimmten Abklingen der Sofortwirkung der besagten kumulierten Dosis auf den besagten Körper nach seiner Entfernung aus der besagten auftreffenden Strahlung oder umgekehrt in Beziehung steht, und Speichern der besagten Bezugssignalhöhe und Abnehmenlassen, der Bezugssignalhöhe nach einer zweiten vorgeschriebenen Abklingfunktion als Reaktion auf eine Verringerung der besagten Meßsignalhöhe, wobei die besagte zweite vorgeschriebene Abklingfunktion mit einem vorbestimmten Abklingen der verzögerten Wirkung der besagten kumulierten Dosis auf den besagten Körper nach Entfernung des besagten Körpers aus der besagten auftretenden Strahlung oder umgekehrt in Beziehung steht, wodurch die Abklinggeschwindigkeit der besagten zweiten vorgeschriebenen Abklingfunktion geringer als die Abklinggeschwindigkeit der besagten ersten vorgeschriebenen Abklingfunktion ist.

19. Verfahren nach Anspruch 18, dadurch gekennzeichnet, daß die besagte zweite vorgeschriebene Dosisfunktion erste und nachfolgende Komponenten umfaßt, wobei die besagte Erstkomponente eine Erstintegrierung mit einer größeren Geschwindigkeit als die besagte erste vorgeschriebene Dosisfunktion bereitstellt, und die besagte Folgekomponente eine nachfolgende Integrierung mit einer geringeren Geschwindigkeit als die besagte erste vorgeschriebene Dosisfunktion bereitstellt.

20. Verfahren nach Anspruch 17, gekennzeichnet durch den Schritt des Anregens der momentanen Erzeugung des besagten sensorisch wahrnehmbaren Signals bei Anlegen von Strom zur Überwachung der besagten ultravioletten Strahlung.

21. Verfahren nach Anspruch 20, gekennzeichnet durch den Schritt, den Meßschritt im wesentlichen während der momentanen Erzeugung des besagten sensorisch wahrnehmbaren Signals zu negieren.

22. Verfahren nach Anspruch 17, gekennzeichnet durch den Schritt des Ausfilterns eines Teils der Strahlung, mit der der Körper belastet ist, damit nur Strahlung im Ultraviolettbereich des elektromagnetischen Spektrums gemessen werden kann.

23. Verfahren nach Anspruch 17, gekennzeichnet durch den Schritt des Anregens der Erzeugung des besagten sensorisch wahrnehmbaren Signals in Abwesenheit des besagten Meßsignals nach Anlegen von Strom zur Überwachung der besagten ultravioletten Strahlung.

24. Verfahren nach Anspruch 23, dadurch gekennzeichnet, daß das besagte sensorisch wahrnehmbare Signal dann momentan erzeugt wird, wenn sowohl der besagte Strom als auch das besagte Meßsignal gleichzeitig schnell an Höhe verlieren oder verschwinden.

## Revendications

1. Un dosimètre pour surveiller l'exposition d'un corps humain à la radiation ultraviolette comprenant un capteur (35) pour détecter ladite radiation frappant un corps humain et pour générer un signal de détection proportionnel à l'intensité de ladite radiation frappant ce corps; et un premier intégrateur (43) adapté pour intégrer ledit signal de détection conformément à une première fonction de dose prescrite pour générer un signal actif représentatif de l'intensité de la dose accumulée de radiation reçue par ledit corps en tenant compte de la tolérance intrinsèque du corps à ladite radiation; caractérisé par un deuxième intégrateur adapté pour intégrer ledit signal de détection conformément à une deuxième fonction de dose prescrite pour générer un signal de référence représentatif de la dose optimale de radiation accumulée à laquelle on désire exposer ledit corps; et un circuit de contrôle (51) pour surveiller l'intensité relative dudit signal actif et dudit signal de référence et générer un signal sensoriellement perceptible lorsque l'intensité du signal actif atteint l'intensité du signal de référence.

2. Un dosimètre selon la revendication 1, caractérisé en ce que ledit premier intégrateur comprend une première mémoire (45) associée pour stocker ladite intensité du signal actif et permettre à ladite intensité du signal actif de diminuer selon une première fonction de décroissance prescrite en réponse à une réduction de ladite intensité du signal de détection, ladite première fonction de décroissance prescrite étant liée à une décroissance prédéterminée de l'effet immédiat de ladite dose accumulée sur ledit corps lorsque ledit corps est soustrait à ladite radiation le frappant ou vice versa, et en ce que ledit deuxième intégrateur comprend une deuxième mémoire (47) pour stocker ladite intensité du signal de référence et permettre à ladite intensité du signal de référence de diminuer selon une deuxième fonction de décroissance prescrite en réponse à une réduction de ladite intensité du signal de détection, ladite deuxième fonction de décroissance prescrite étant liée à une décroissance prédéterminée de l'effet retardé de ladite dose accumulée sur ledit corps lorsque ledit corps est soustrait à ladite radiation le frappant ou vice versa, de sorte que le taux de décroissance selon ladite deuxième fonction de décroissance prescrite est inférieur au taux de décroissance selon ladite première fonction de décroissance prescrite.

3. Un dosimètre selon la revendication 1, caractérisé en ce que ladite deuxième fonction de dose prescrite comprend des composantes initiale et subséquente, ladite composante initiale fournissant une intégration initiale à un taux supérieur à ladite première fonction de dose prescrite, et ladite composante subséquente fournissant une intégration subséquente à un taux inférieur à ladite première fonction de dose prescrite.

4. Un dosimètre selon la revendication 1, caractérisé en ce que ledit premier intégrateur (43) comporte un sélecteur comprenant une première résistance pour sélectionner différentes premières fonctions de dose prescrite selon la tolérance dudit corps avec lequel ledit dosimètre doit être utilisé.

5. Un dosimètre selon la revendication 2, caractérisé en ce que ladite première mémoire (45) comprend un premier condensateur (C1) et ladite première fonction est déterminée par des premières résistances (R3-R7) couplées en série avec ledit condensateur (C1) pour former un premier circuit de charge R-C en série.

6. Un dosimètre selon la revendication 5, caractérisé en ce que ledit premier intégrateur (43) comporte un sélecteur pour sélectionner différentes premières fonctions de dose prescrite selon la tolérance dudit corps avec lequel le dosimètre doit être utilisé, ledit sélecteur comprenant une pluralité de premières résistances (R3-R7) de valeurs différentes et un commutateur (60) pour coupler sélectivement une première résistance unique (R3-R7) audit condensateur (C1) à tout moment.

7. Un dosimètre selon la revendication 3, caractérisé en ce que ladite deuxième mémoire (47) comprend un deuxième condensateur (C2) et ladite composante initiale de ladite deuxième fonction de dose prescrite est déterminée par un condensateur de dérivation (C4) couplé en série audit deuxième condensateur (C2) et la composante subséquente de ladite deuxième fonction de dose prescrite est déterminée par une deuxième résistance (R9) couplée en série avec ledit troisième condensateur (C3) et en parallèle avec ledit condensateur de dérivation (C4).

8. Un dosimètre selon la revendication 7, caractérisé en ce que ledit premier condensateur (C1) et ledit deuxième condensateur (C2) sont disposés de sorte que lesdites première et deuxième fonctions de dose prescrite sont déterminées par la fuite naturelle dudit premier condensateur (C1) et dudit deuxième condensateur (C2) respectivement.

9. Un dosimètre selon la revendication 7, caractérisé en ce que ladite deuxième mémoire (47) est dotée d'un moyen de réinitialisation pour y effacer ladite intensité du signal de référence lorsque ledit corps est soustrait à la ladite radiation le frappant ou vice versa.

10. Un dosimètre selon la revendication 6, caractérisé en ce que ladite deuxième mémoire (47) comprend un deuxième condensateur (C2) connecté audit deuxième sélecteur pour des premières fonctions de dose prescrite spécifiques pour fournir un chemin de décharge quasi instantanée audit deuxième condensateur lorsque ledit corps est soustrait à ladite radiation le frappant ou vice versa.

11. Un dosimètre selon la revendication 1, caractérisé en ce que ledit circuit de contrôle (51) comprend un transducteur (55) pour générer ledit signal sensoriellement perceptible, et un commutateur électronique (52) pour activer ledit transducteur lorsque ladite intensité du signal actif atteint ladite intensité du signal de référence.

12. Un dosimètre selon la revendication 1, caractérisé en ce que ledit circuit de contrôle comprend un circuit de déclenchement (59) pour inciter la génération dudit signal sensoriellement perceptible momentanément à la mise sous tension dudit dosimètre pour indiquer que ledit dosmètre est opérationnel.

13. Un dosimètre selon la revendication 12, caractérisé en ce que ledit circuit de contrôle comprend un comparateur (53) aux entrées duquel sont couplées la sortie du signal actif et la sortie du signal de référence et en ce que ledit circuit de déclenchement comprend un condensateur de commande couplé entre une source d'alimentation pour le dosimètre et la sortie du signal actif dudit premier intégrateur, de sorte qu'à la mise sous tension dudit dosimètre, ladite intensité du signal actif va atteindre rapidement ladite intensité du signal de référence puis revenir à une intensité nominale spécifiée par ledit premier intégrateur après que ledit condensateur de commande se soit complètement chargé.

14. Un dosimètre selon la revendication 12, caractérisé en ce que ledit capteur comprend un moyen de retard pour quasiment inverser logiquement sa fonction durant la génération momentanée dudit signal sensoriellement perceptible.

15. Un dosimètre selon la revendication 1, caractérisé par un autre circuit de déclenchement adapté pour inciter la génération dudit signal sensoriellement perceptible en l'absence dudit signal de détection après la mise sous tension dudit dosimètre.

16. Un dosimètre selon la revendication 15, caractérisé en ce que ledit autre circuit de déclenchement incite la génération dudit signal sensoriellement perceptible momentanément lorsque ledit signal de puissance et ledit signal de détection perdent simultanément de l'intensité ou disparaissent.

17. Une méthode pour surveiller l'exposition d'un corps à la radiation ultraviolette comprenant les étapes de:
détection de la radiation ultraviolette frappant un corps;
génération d'un signal de détection proportionnel à l'intensité de ladite radiation frappant ce corps;
réalisation d'une première intégration dudit signal de détection selon une première fonction de dose prescrite pour fournir un signal actif représentatif de la dose de radiation accumulée reçue par ledit corps en tenant compte de la tolérance intrinsèque de ce corps à ladite radiation; caractérisée par
la réalisation d'une deuxième intégration dudit signal de détection selon une deuxième fonction de dose prescrite pour fournir un signal de référence représentatif de la dose optimale de radiation accumulée auquel on désire exposer ledit corps;
la surveillance de l'intensité relative dudit signal actif et dudit signal de référence; et
la génération d'un signal sensoriellement perceptible lorsque ladite intensité du signal actif atteint ladite intensité du signal de référence.

18. Une méthode selon la revendication 17, caractérisée par l'étape de mémorisation de ladite intensité du signal actif devant diminuer selon une première fonction de décroissance prescrite suite à une réduction de ladite intensité du signal de détection, ladite première fonction de décroissance prescrite étant associée à une décroissance prédéterminée de l'effet immédiat de ladite dose accumulée sur ledit corps lorsqu'il est soustrait à la radiation le frappant ou vice versa, et de mémorisation de ladite intensité du signal de référence, en permettant à l'intensité du signal de référence de diminuer selon une deuxième fonction de décroissance prescrite suite à une réduction deladite intensité du signal de détection, ladite deuxième fonction de décroissance prescrite étant liée à une décroissance prédéterminée de l'effet retardé deladite dose accumulée sur ledit corps lorsque ledit corps est soustrait à la radiation le frappant ou vice versa, de sorte que le taux de décroissance de ladite deuxième fonction de décroissance prescrite est inférieur au taux de décroissance de ladite première fonction de décroissance prescrite.

19. Une méthode selon la revendication 18, caractérisée en ce que ladite deuxième fonction de dose prescrite comprend des composantes initiale et subséquente, ladite composante initiale fournissant une intégration initiale à un taux supérieur à ladite première fonction de dose prescrite, et ladite composante subséquente fournissant une intégration subséquente à un taux inférieur à ladite première fonction de dose prescrite.

20. Une méthode selon la revendication 17, caractérisée par l'étape d'incitation de la génération dudit signal sensoriellement perceptible momentanément à la mise sous tension pour surveiller ladite radiation ultraviolette.

21. Une méthode selon la revendication 20, caractérisée par l'étape de quasi-inversion logique de l'étape de détection durant la génération momentanée dudit signal sensoriellement perceptible.

22. Une méthode selon la revendication 17, caractérisée par l'étape de filtrage d'une partie de la radiation à laquelle le corps est exposé pour ne laisser que la radiation dans la région ultraviolette du spectre électromagnétique à détecter.

23. Une méthode selon la revendication 17, caractérisée par l'étape d'incitation de la génération dudit signal sensoriellement perceptible en l'absence dudit signal de détection après la mise sous tension pour surveiller ladite radiation ultraviolette.

24. Une méthode selon la revendication 23, caractérisée en ce que le signal sensoriellement perceptible est momentanément généré lorsque ledit signal de puissance et ledit signal de détection perdent simultanément rapidement de l'intensité ou disparaissent.
